# EUROPEAN PATENT APPLICATION

(11) **EP 3 241 494 A1**
(43) Date of publication of application: **08.11.2017**
(21) Application number: 16168368.5
(22) Date of filing: 04.05.2016
(51) Int. Cl.: A61B 5/0428

(54) **DEVICE FOR DETECTING OR MONITORING BIOELECTRICAL PARAMETERS**

(71) Applicant: Valtronic Technologies (Holding) SA, 1343 Les Charbonnières (CH)
(72) Inventor: Lepple-Wienhues, Albrecht, F-25300 Pontarlier (FR)
(74) Representative: Patentanwälte Ruff, Wilhelm, Beier, Dauster & Partner mbB

(57) **Abstract**

The invention refers to a device (11) for detecting or monitoring electrical parameters in a human subject or animal subject by measuring electrical potentials. According to the invention this device comprises at least one electrical connection attachable to tissue of said human or animal subject, serving as a material electrode (5), and at least one electronic circuitry and/or at least one microprocessor/microcontroller and, where appropriate, software, creating a node serving as a virtual reference electrode (7). According to the invention said node can be held at an electrical reference potential by said circuitry and/or microprocessor/microcontroller. Further, the inventive device comprises means to measure electrical potential changes between said electrical reference potential of the node and the electrical potential of the connection at the tissue of said human or animal subject.

## Description

### FIELD OF THE INVENTION

This invention relates to a device and to a method for detecting or monitoring electrical parameters in a human subject or animal subject. Further, this invention relates to an item of personal use to be worn by a human subject or animal subject.

### BACKGROUND OF THE INVENTION

Devices for detecting or monitoring bioelectrical parameters are known from the prior art. Traditionally, the circuit topology for measuring bioelectrical signals consists of a low noise instrumentation amplifier, filters, an analog-to-digital converter, and a digital signal processor or microcontroller. Typical examples for such devices are recording devices for electrocardiogram (ECG), electroencephalography (EEG), and electromyography (EMG), or corresponding wearable clinical heart monitors. In the further discussion of the prior art, an ECG recorder or monitor will be used as an example.

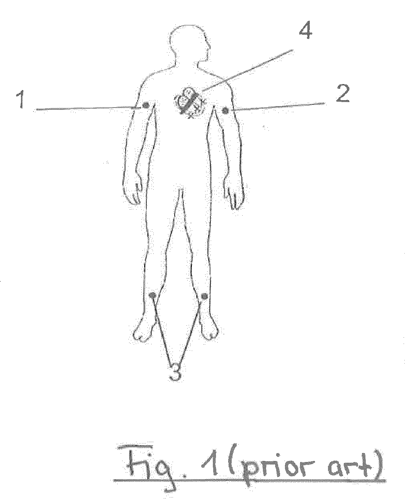

In the recording and/or monitoring devices for ECG, known from the prior art, the above-mentioned instrumentation amplifier using low noise operational amplifiers amplifies the differential ECG signal derived from two or more electrodes attached to the patient's body. In this context, both mobile and stationary recording devices use at least two electrodes spaced at a considerable distance of more than several centimetres at the patient's body. In this respect, body parts like extremities or limbs can be used like conductors. This fact will be explained later in further detail in context with Fig. 1 illustrating the prior art.

This requirement of at least two electrodes spaced at a considerable distance on the patient's body represents a major hurdle when trying to miniaturize a corresponding recording device, e.g. as a wearable ECG recording patch, wristband, earbud and the like. The reason is that a minimum of two distant electrodes must be connected to the device by conductors, consisting of cables, extensions, etc. However, miniaturizing such devices is highly desirable to detect and monitor vital parameters as ECG, pulse wave velocity etc. for a long period.

In this context, it has to be mentioned that especially cardiovascular diseases represent a major health concern and cause of death globally.

Two examples of cardiovascular conditions associated with frequent disease and death are cardiac arrhythmias and arterial hypertension.

Cardiac arrhythmias, also known as cardiac dysrhythmias, are quite frequent e.g. in patients with cardiac insufficiency and can lead to severe complications like stroke, embolism, cardiac fibrillation and death. Quite often such arrhythmias cannot be felt by the patient, can be transitory and go unnoticed until severe complications arise. ECG monitoring can alert patients and caretakers about arrhythmic conditions and timely therapy can avoid severe complications.

The major cardiovascular risk factor is arterial hypertension as the other example of said cardiovascular conditions leading to heart failures, strokes, kidney and eye diseases, and reduced life expectancy.

Therapeutic blood pressure control including efficient drug treatment decreases the risk of complications. It is therefore important to identify persons with elevated blood pressures and monitor their blood pressure information on a regular basis.

In this context, a problem lies in the fact that arterial blood pressure (BP) varies widely with daytime, activity level, excitation, alertness and other factors. Therefore, singular measurements of BP are helpful, but frequently misidentify subjects either as hypertensive or miss to identify hypertensive subjects. E.g. the well-known white-coat syndrome describes elevated BP readings induced by the excitement of the procedure. An important marker for cardiovascular risk is the absence of nightly BP decline during sleep.

Therefore, ambulant blood pressure monitoring is preferable and has a higher potential to correctly diagnose arterial hypertension. However, existing blood pressure devices and ECG recording devices are often clumsy, uncomfortable, difficult to use, prone to user error, cannot monitor continuously, and are neither practical nor acceptable for routine monitoring while sleeping. Clumsiness also leads to reducing patient compliance in ambulant BP monitoring.

In this context, pulse wave velocity is considered to have the best predictive value for cardiovascular outcome. Furthermore, pulse wave velocity is closely correlated to arterial blood pressure. For a given subject, it is possible to transform measured pulse wave velocity into blood pressure values, using an individual calibration. However, to provide a wearable pulse wave velocity detecting and monitoring device, a miniaturized ECG recording unit is a prerequisite when using the ECG signal as a measure of the starting time of the pulse wave.

In the prior art, US-B2-9,237,848 describes a patch including first and second electrodes located at opposite ends of the patch and including a flexible connecting member as part of a wireless physiological sensing device. The device is used for recording a single lead ECG. The two electrodes are separated by the entire length of the patch in order to record a differential potential from the body surface.

US-B2-9,078,577 describes a circuit for heartbeat detection and beat timing extraction. In order to create a digital timer signal at the moment of the ECG-R-spike, a bandpass filter and a low-pass filter are connected to a comparator. US-patent 9,078,577 uses a differential preamplifier connected to two skin electrodes that are spaced at a distance in order to amplify a voltage difference between those two electrodes. Furthermore, several electronic means are described in order to allow for reversed electrode connection of the two electrodes resulting in a polarity change of the recorded signal. The requirement for two separate electrodes which are spaced at a distance of several centimetres on the patient's body and which are connected by cables according to US-patent 9,078,577 is also illustrated in another publication of the inventors of US-patent 9,078,577, namely in IEEE Transactions on Biomedical Circuits and Systems, vol. 9, no. 3, June 2015, pages 370-376, David Da He and Charles G. Sodini, A 58 nW ECG ASIC With Motion-Tolerant Heartbeat Timing Extraction for Wearable Cardiovascular Monitoring. The recording of an ECG from the ear-neck-area is illustrated in Figs. 10d and 11 of this publication using two separate electrodes.

As a consequence, the system described in US-patent 9,078,577 also shows the features of the prior art discussed earlier, namely the requirement of at least two electrodes spaced at a considerable distance on the patient's body.

US-B2-8,086,301 describes a method and apparatus for cufflessly and non-invasively measuring wrist blood pressure including detecting feature points from an electrocardiogram. The device comprises an ECG electrode integrated in an apparatus and a second electrode, where an ECG signal is measured by contact of a finger of the other hand, which is free from the apparatus in order to close the circuitry. Again, the feature of the prior art is the requirement of at least two electrodes spaced at a distance, where the second arm free from the apparatus is used as a conductor to a potential area opposite of the heart.

### OBJECT OF THE INVENTION

The present invention has the object to overcome the described limitations of the discussed prior art. The invention shall provide a device recording bioelectrical parameters which is capable to detect and monitor the necessary parameters, in particular continuously, and which will not hinder the user to live his/her normal life or hinder the user only to an acceptable small or very small extent.

### SUMMARY OF THE INVENTION

For solving this objective and other objectives, the present invention provides a device as mentioned above, as defined in independent claim 1. Preferred embodiments of this device are defined in the claims dependent from claim 1. The invention also provides an item of personal use as defined in independent claim 11.

Further, the present invention provides a method for detecting or monitoring electrical parameters in a human subject or animal subject, in particular by using the inventive device, as defined in independent claim 12. Preferred embodiments of this inventive method are shown in the method claims dependent from claim 12.

All features of all claims are hereby incorporated into the description by explicit reference to the claims.

### DETAILED DESCRIPTION OF THE INVENTION

The invention provides a device for detecting or monitoring electrical parameters in a human subject or animal subject by measuring electrical potentials, comprising at least one electrical connection or lead, at least one electronic circuitry and/or at least one microprocessor/microcontroller (and, where appropriate, software), and means to measure electrical potential changes.

According to the invention, the electrical connection is attachable to tissue of said human or animal subject, and serves as a material electrode. The electronic circuitry and/or microprocessor/microcontroller creates a node which serves as a virtual reference electrode, wherein said node can be held at an electrical reference potential by said circuitry and/or microprocessor/microcontroller. The means to measure electrical potential changes are provided to measure those changes between said electrical reference potential of the node and the electrical potential of the connection at the tissue of said human or animal subject.

According to the invention, the term "material electrode" is used in the sense of an electrical contact area between tissue, i. e. a body surface of a human or an animal and the electrical connection, i. e. the inventive device. Further, according to the invention, the term "virtual reference electrode" is used in the sense of a virtual ground or reference electrode, created by the electronic circuitry and/or microprocessor/microcontroller. As a consequence this "reference electrode" is indeed virtual and not material (i. e. it is not created by a physical contact of an electrical connection with tissue of a human or animal subject).

This essential advantage of the inventive device as defined in claim 1 of the present patent application can be also explained as follows.

As described above, the inventive device includes a least one electrical connection or lead attachable to tissue, i. e. it includes at least one material electrode. Since a recording of a bioelectrical signal is per definition measuring of a charge movement or electrical potential difference, a minimum of two electrical nodes or points is required. In order to avoid the disadvantages of the prior art, as described earlier, in the present invention the necessary second electrical node or point is created inside the electronic circuitry and/or microprocessor/microcontroller. This reference potential node serves as a virtual ground or reference electrode. As a consequence, the need of the prior art to provide a second (material/physical) electrode, with the need for a cabbling to a second (distant) tissue area of a human or animal body is eliminated.

According to the invention, the term "tissue" is every association of cells, up to a complete organ. Preferably said tissue is a skin or mucous membrane.

The inventive device, together with its advantages can be further describes using ECG (electro cardiogram) as an example, although a similar description could be made for any other bioelectrical recording.

According to the invention, in an ECG, the virtual electrode node will be permanently held on the potential of the base line of the ECG. The electronic circuitry (and/or the microprocessor/microcontroller) will avoid the potential of the virtual electrode to follow the potential changes during the typical phases of an ECG. E. g., the phases in an ECG are labeled with "p q r s t" describing deflections of a typical skin electrode potential. To this end, the electronic circuitry (and/or microprocessor/microcontroller) will minimize or avoid any charge change of that node during said deflections of the ECG signal. In other words, the circuitry (and/or microprocessor/microcontroller) will hold said node at a steady resting potential and will avoid the potential of said node to follow the potential changes of ECG signal deflections, particularly the "p q r s t" deflections, specifically the large deflections using the "qrs" complex.

Since a single electrode (connected to tissue, e. g. skin) will show potential deflections during the ECG cycle, a potential difference between the single skin electrode and the virtual reference electrode can be measured against the virtual reference node reflecting the potential changes on the skin contacted area during the ECG cycle. Therefore (to emphasize again) the need for a (material) reference electrode connected to the body surface is eliminated.

In other words, the electronic circuitry (and/or microprocessor/microcontroller) creates a virtual reference node that allows to separate deflection intervals in the ECG signal from baseline intervals in the frequency and time domains, in contrast to the prior art that differentiates ECG deflection from baseline spatially, using at least two distant (material) electrodes on the body surface.

All these aspects will be further discussed in the description of the drawings, also explaining the inventive method in further detail.

In a preferred embodiment of the invention, the inventive device, in particular said electronic circuitry or said microprocessor/microcontroller comprises at least one capacitor separating said electrical connection and said node. In other words, this capacitor is placed between the material electrode and the virtual reference node.

In accordance with the earlier explanation, in the corresponding embodiment with a capacitor, the voltage across that capacitor will change during the ECG cycle, thus reflecting the potential changes of the material electrode (e. g. skin electrode) during the deflections of the ECG signal. The reference node will be kept on the average baseline potential by electronic means.

In this context, it is further preferred according to the invention that the electronic circuitry or the microprocessor/microcontroller comprises filters and/or switches for distributing signals resulting from electrical potential changes. In particular these filters and/or switches are provided for distributing signals between the terminal (end) of the capacitor which is assigned to the electrical connection and the terminal (end) of the capacitor which is assigned to the node.

Therefore the electronic means to keep the reference node on the average baseline potential can comprise in preferred embodiments frequency filters, specifically low pass filters, a time domain predictor switch connecting the reference point to the baseline section exclusively during the baseline phases of the signal, a switch triggered by flank steepness, or a combination thereof.

In another preferred embodiment said filters and/or said switches are provided in pairs. This can also be seen in the figures accompanying this description.

The filters and/or switches can be realized in preferred embodiments as analog and/or digital filters and switches. In a preferred embodiment said filters and switches will be combined. In another preferred embodiment a computing unit, e. g. a microcontroller, will control and combine filters and switches to optimize timing extraction from e. g. the ECG signal, signal to noise ratio and signal stability.

As already indicated it is preferred according to the invention that the change of potential of the material/physical electrode, e. g. the skin electrode, versus the virtual reference node can be measured as a voltage and/or as a charge movement and/or as a current.

In one preferred embodiment, a resistor and a capacitor are connected in series between the material electrode and the virtual reference node. Any charge movement towards said capacitor will be detected using a volt meter measuring the voltage drop across the resistor. Said voltage drop will reflect the difference in potential between the material electrode and the virtual reference node.

In all preferred embodiments, the voltage across said capacitor can be measured, e. g. using a differential operational amplifier. In another preferred embodiment, the charge or current flowing between the material electrode and said capacitor will be measured.

It is further preferred according to the invention, that only one electrical connection to tissue of said human or animal subject is provided. Therefore, there is only one electrical connection, i. e. only one material/physical electrode at the tissue of said human or animal subject.

In all embodiments of the present invention, in particular also in all embodiments comprising only one electrical connection to the tissue of said human or animal subject, it is preferred that the corresponding electrical connection comprises at least two contact points or contact areas. In this context, the terms "contact points" and "contact areas" are meant to describe an electrode that has more than one resistive or capacitive electrical connection to tissue. This definition is also valid in cases where there is only one electrical connection of the device to said tissue. This fact will be explained here using an adhesive skin electrode as an example. Such electrodes typically consist of a metallic conductive surface touching the skin and being surrounded by adhesive foil. The corresponding conductive surface may be divided into several segments, that may be elastically held in order to allow a better contact with the contour of the skin. Those multiple contact points or contact areas are meant to reduce changes in resistance and/or capacitance between the electrode and the tissue, e. g. due to movement, local separation, sweating etc. In spite of the fact that there are multiple contact points/contact areas, all these embodiments still provide only one electrical connection to the tissue, i. e. only one material/physical electrode at the tissue of the corresponding human or animal subject.

According to the described preferred embodiments said contact points or contact areas are provided at a comparably low distance of between at least 0,1 mm and at most 45 mm. Typically and preferred said multiple contact points/contact areas are even closer together with a distance between 5 mm and 25 mm, in particular between 10 mm and 15 mm.

To emphasize again, multiple contact points or multiple contact areas with the preferred small distances as mentioned above, are considered to be one single electrode according to the present invention. Those contact points/contact areas are not used to measure potential differences between different body surface regions, but instead are used in order to reduce vulnerabilities due to resistance/contact changes of the corresponding contact points/areas with the body surface and/or reduce noise from unwanted bioelectrical origins like electromyographic potentials.

In preferred embodiments, the inventive device further comprises at least one data transmitter/data receiver. This term means any electronic component receiving data from outside the device and/or for transmitting data from the device. Normally such a component of the inventive device will include an antenna. Preferably, the data transmitter/data receiver is a wireless data transmitter/data receiver making the device independent from any wire connection to a further component.

According to a further preferred embodiment, the inventive device further comprises at least one power source. The term "power source" means any device or equipment capable to deliver energy. Preferably, the power source is a galvanic element, in particular a battery.

According to a further preferred embodiment, the inventive device further comprises at least one illumination source. The term "illumination source" means any device or equipment which is capable to serve as a source of electromagnetic radiation, in particular of electromagnetic radiation visible to the human eye.

According to a further preferred embodiment, the inventive device further comprises at least one photodetector. The term "photodetector" means any device or equipment to serve as a sensor for electromagnetic radiation, in particular visible electromagnetic radiation.

In other preferred embodiments of the present invention, the inventive device further comprises a temperature sensor. Therefore, the temperature of the tissue can be considered in monitoring the corresponding parameters in particular those related to cardiovascular conditions or diseases.

In other preferred embodiments of the present invention, the inventive device further comprises a sensor or sensor unit to measure the electrical impedance of the tissue, in particular of the skin and/or the mucous membrane. Electrical impedance is a further parameter which can be considered in the evaluation of the data measured by the inventive device.

According to the invention, it is further preferred that the inventive device is formed as or is integrated in an item of personal use to be worn by the human subject or animal subject.

According to the invention, the term "item of personal use" shall include any item which is capable to be in contact to tissue of the corresponding human subject or animal subject during daily life. As a consequence, the term "to wear/worn" shall mean that this item of personal use is at the body or in the body of the corresponding subject in contact with the corresponding tissue. The term "item of personal use" will be further explained later in context with the corresponding claims directed to said item of personal use.

Preferably, the inventive device has a separate enclosure housing at least part of the components of the device, in particular all of the components of the device. In these preferred embodiments, the design of the enclosure preferably is adapted to the design of a normal item of personal use so that the inventive device is a substitute for this normal item.

According to preferred embodiments of the inventive device, the components of the inventive device (mandatory and/or additional) are provided on at least one outer surface of the enclosure or of the item. In this context, it is further preferred that these components of the inventive device are provided on at least one outer surface of the enclosure. As a consequence, the most important components of the device for detecting and monitoring the corresponding parameters can be close to the corresponding tissue of the human or animal subject.

With the embodiments mentioned above, it is further preferred that the corresponding components are provided on an outer circumferential surface of the item, in particular the enclosure. Preferably, this outer circumferential surface is an outer shell surface of a cylinder or an outer shell surface of a cone or an outer shell surface of a truncated cone.

According to the invention an inventive device is further preferred, wherein the electrical parameters detected or monitored in a human subject or animal subject are related to cardiovascular conditions or are related to cardiovascular diseases.

According to the invention the terms "cardiovascular conditions" and "cardiovascular diseases", respectively, shall include all abnormal conditions related to the heart or to the blood vessels of a human or animal subject. All parameters related to those conditions can be monitored according to this invention, not only in the course of therapy, but also in the course of prevention.

In addition to the inventive device as described, the invention also provides an item of personal use to be worn by a human subject or animal subject. This inventive item of personal use is characterized in that the inventive device as described earlier, forms this item or is integrated into this item. In this context, it is referred to the above definition of an "item of personal use".

According to the invention, it is preferred that the item of personal use is an earplug or hearing aid, which can be brought in contact to any tissue of any part of the ear. Preferably, in such an item, the item, in particular the enclosure of the item is designed to fit within at least a part of the ear, in particular with the (outer) ear canal.

Further, it is preferred according to the invention that the item of personal use is a jewelry, in particular an ear clip, an ear ring, a wrist watch, a bracelet, a necklace or a piercing. Therefore, such kind of item is in close contact with the skin of the corresponding subject.

Further, it is preferred according to the invention that the item of personal use is an eye glass frame or a band, in particular a headband. Therefore, these items are also in close contact to the skin of the corresponding subject. If the item is a band it is preferred that the item is an elastic band establishing close contact to the skin due to its elasticity.

Further, it is preferred according to the invention that the item of personal use is a denture, a dental implant, a dental crown, a dental bridge, an onlay or an inlay. Therefore, these items are in close contact to the mucosa of the mouth.

Further, it is preferred according to the invention that the item of personal use is a piece of clothing, in particular a piece of underclothing. Therefore, these items are in close contact to the skin of the corresponding subject.

Finally, the invention discloses an inventive method for detecting or monitoring electrical parameters in a human subject or animal subject, in particular by using the inventive device as already described. According to this inventive method at least one electrical connection is attached to tissue, in particular to skin or mucous membrane of said subject. This electrical connection serves as a material electrode. Further, in this method a node serving as a virtual reference electrode is created by at least one electronic circuitry and/or at least one microprocessor/microcontroller, and, where appropriate software, wherein said node can be held at an electrical reference potential by said circuitry and/or microprocessor/microcontroller. Finally, in this inventive method, electrical potential changes are measured between said electrical reference potential of the node and the electrical potential of the connection at the tissue of said human or animal subject.

In a preferred embodiment, said electrical potential changes in the inventive method are measured as a voltage change and/or as a charge movement and/or as a current.

In another preferred embodiment of the inventive method, at least one capacitor, in particular at least one resistor and at least one capacitor connected in series, are used for the measurement of electric potential changes, wherein preferably a charge movement towards that capacitor is detected measuring the voltage drop across the resistor.

As it is preferred that the inventive method uses the inventive device, it is referred to the above description for further details in describing the inventive method.

Further, the following features and embodiments are important and advantageous in relation to the products and methods according to the invention.

Several methods to reliably detect the onset of ventricular depolarization resulting in the qrs complex with its r spike within a noisy recording are known to the specialist. These methods are e.g. used in detecting the fetal heartbeat using fetal ECG electrodes, where the heartbeat of the fetus has to be detected within a background of maternal ECG and uterine and other muscle myographic signals. Autocorrelation, Teager Energy Operator, and power spectral density analysis, as well as frequency filtering belong to the methods in use for those purposes. Using such methods it is possible to reliably detect the onset of ventricular depolarization.

In a further embodiment, since the method or the device disclosed will continuously record electrical heartbeat (ventricular depolarization), a detection algorithm can be implemented in the device in order to detect arrhythmia or other pathologic states. The device can then give a warning to the patient and/or caretaker, e.g. an acoustical or optical warning, or it can transmit data to another device. This is especially useful in paroxysmal arrhythmia, e.g. supraventricular tachycardia due to atrial fibrillation. This is a frequent heart disease that has a high risk of stroke and embolia and is difficult to detect with traditional diagnostic methods, because it is often intermittent and may not be felt by the patient but missed during point-of-care ECG recordings.

According to the invention, a method is preferred wherein also the varying hemoglobin concentrations in tissue are recorded, in particular simultaneously, for determining the arrival of the arterial pressure pulse and then the corresponding values of the actual pulse wave velocity of the subject are calculated.

The hydraulic pulse wave onset is determined with a setup similar to reflective pulse-wave oximetry. One or multiple light-emitting diodes (LEDs) are emitting light into the skin adjacent to the device. One or multiple photodiodes or phototransistors is/are measuring reflected light. Typical LED wavelengths are 660 nm visible or 940 nm infrared. Absorption and reflection of light at these wavelengths allows determining the concentration of hemoglobin in the tissue, even discriminating between oxygenated and deoxygenated hemoglobin. A suitable illumination and readout pattern setting corrects for ambient light contamination. A fluctuating signal for hemoglobin concentration in the tissue is obtained, enabling the determination of the arrival time of the arterial pressure pulse. Because the onset of cardiac concentration is available from the ECG recording and the distance through the vascular tree between heart and device is known the device can thus calculate the individual, actual pulse wave velocity.

In addition, it is further preferred with the inventive method to transform the values of pulse wave velocity into blood pressure values (BP values), using conversion factors provided by a calibration performed on the corresponding subject, in particular the corresponding individual subject.

In another preferred embodiment the device will comprise means for automated calibration. For a given subject, it is possible according to the inventive method to transform measured pulse wave velocity into blood pressure values, using an individual calibration. Calibration could be performed by a caretaker taking traditional BP readings during an ergometer session simultaneously with PWV readings, thus creating the individual calibration parameters for a given subject. These parameters can then be transferred and stored in a device. Conversion or transformation of PWV values into BP values has the advantage that caretakers are more accustomed to evaluate BP values and the thresholds for therapeutic intervention are well known and established. A calibration procedure can be facilitated by software on the device as well as by algorithms in other devices like computing devices and/or a blood pressure monitor. A calibration could be performed in a physician's office or in the user's home environment. For calibration a series of PWV measurement will be synchronized in time with simultaneous BP measurements. After determination of stable baseline values the subject will be asked to perform physical exercise in order to take readings at elevated pulse rates and BPs, as well as in the recovery phase. In addition, the geometrical vascular tree distance of the device being worn at a particular body part can be measured and calculated, e.g. using a tape measure. Using these data an algorithm can determine a precise individual correlation between PWC readings and BP of a given subject, enabling the device to calculate BP values from PWV readings for this subject and present those values to the patient as well as to caretakers.

In another embodiment the device will have algorithms implemented that will track the pulse wave patterns and pulse rate patterns in combination with temperature and skin resistance readings for identifying patterns typical for a given subject. This pattern recognition can then be utilized for biometric patient recognition and data safety. This device can encrypt data and verify patient identity, thus avoiding misidentification of patient data as well as abuse of patient data in compliance with protocols like e.g. HIPAA (the Health Insurance Portability and Accountability Act) setting the standard in USA for protecting sensitive patient data.

In another embodiment temperature and skin resistance are additional parameters the device will record for the detection of e.g. physical work conditions that naturally come with increased BP and heartbeat frequencies. Thus, the device can avoid warning about physiologically elevated BP, while its capability to detect pathological changes of PWV or BP Is increased. Furthermore, cardiac pathological events that lead to generalized symptoms can be detected, e.g. cardiac infarction or severe arrhythmias, and local or remote warnings / alerts can be performed.

In another embodiment the device can record and thus learn typical parameter bands for a given patient and automatically adjust alarm thresholds for pathologic conditions. In another embodiment those thresholds can be manually set by the caretaker, doctor or patient.

In another application, the hemodynamic parameters measured by the disclosed invention will be usable to control, assess and improve the training status of patients in rehabilitation as well as athletes. The cardiovascular parameters measured here greatly increase the information level about cardiac health and training status over existing training aids.

To summarize, the present invention discloses a method and device for a non-invasive, miniaturized, wearable cardiovascular risk information monitor where many disadvantages of the prior art are eliminated. The disclosed miniaturized, integrated wearable device will be practical and acceptable because of minimal interference with daily lifestyle. The disclosed device will be acceptable and not interruptive during sleep. The disclosed device will integrate in a barely noticeable wearable, e.g. an ear plug, or integrate into a device already worn like hearing aid or eye glasses.

The disclosed device will obtain a long term monitoring of cardiovascular risk parameters and even allow the computation and display of BP values for a given subject.

Further advantages and features of the invention will become clear from the following description of the drawings in the conjunction with the dependent claims. The individual features can be realized either singly or separately in combination in one embodiment of the invention. The drawings merely serve for illustration and better understanding of the invention and are not to be understood as in any way limiting the invention.

The figures schematically show:
- Fig. 1: an illustration of the electrical connections used for a classical prior art electrocardiogram, and
- Fig. 2: an illustration of the present invention with the figures 2A to 2B.

Fig. 1 schematically illustrates the electrical connections or leads used in classical prior art electrocardiography. As explained earlier, in the prior art, electrocardiography monitoring is performed by measuring electrical potential differences between a material ground electrode and electrodes which are attached to parts of the body as far apart as possible. This is done to maximize the voltage readout created by the depolarization front passing the myocard.

This situation in classical ECG (electrocardiography) is illustrated in Fig. 1 by the electrodes 1, 2 and 3 (arranged as far apart as possible) and by the electrical depolarization front traveling through the myocard (reference number 4 in Fig. 1).

As a consequence, it is evident from Fig. 1 that the largest electrical potential differences will be recorded by electrodes that are spaced in a way that much of the depolarization front is located in-between a given electrode pair.

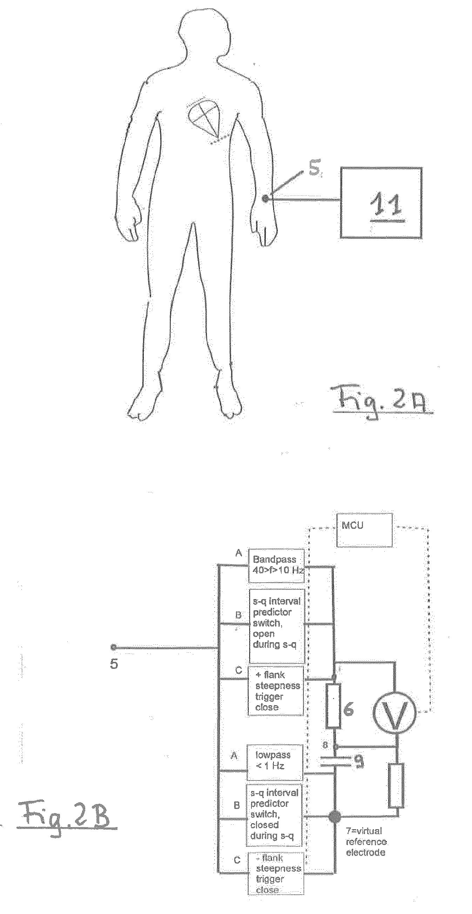

In Fig. 2A the device 11 is connected to the patients wrist using one single skin electrode 5. In other preferred embodiments, the device 11 can be connected to either surface area of the body instead. The device is not depicted to scale but magnified schematically.

Fig. 2B illustrates schematically the circuitry of the device 11 according to one preferred embodiment. A capacitor 9 separates the material electrode 5 and the virtual reference (electrode) node 7. Several electronic filters and / or switches connect the material electrode 5 with either the deflection node 8 or the virtual reference node 7 in a time and / or frequency dependent manner. The voltage between the deflection node 8 and the virtual reference node 7 will variably charge said capacitor and will represent the ECG signal that can be further analysed.

In an embodiment A the filters and / or switches to distribute the signal between the "r" spike side of capacitor 9 and the "reference electrode" side of capacitor 9 comprise a bandpass filter from 10 to 40 Hz feeding into the "r" spike side of capacitor 9. A low-pass filter with a cutoff frequency of <1 Hz feeds the "reference electrode" side of capacitor 9.

In an embodiment B a switch is activated by a periodic qrs "predictor/timer", since the variance of the beat-to-beat time is small except in the case of arrhythmia. Said switch will connect the body surface electrode 5 during baseline intervals, e.g. "st" and/ or "tq", with the "reference electrode" side of capacitor 9. During the "qrs" and / or "p" and /or "t" deflection interval said switch will connect the body surface electrode 5 to the "R"spike side of capacitor 9.

In an embodiment C said switch is activated by a flange detector or derivative detector switching between both sides of capacitor 9. The flange detector will detect the change of the signal upon beginning of deflections "p" and / or "qrs" and or "t"" and actuate the switch, again connecting the body surface electrode 5 to the "reference electrode" side of capacitor 9 only during "baseline" intervals, while at the deflection intervals the connection to the "R"spike side of capacitor 9 in combination with means to measure voltage and / or charge flow will allow for a measurement of the ECG deflections against a virtual reference potential inside the circuitry.

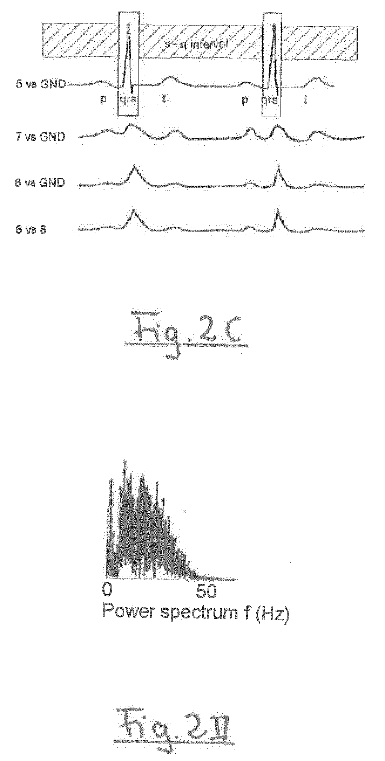

Fig. 2C illustrates the idealized potential differences between the different nodes in Fig. 2B in the time domain. "GND" designates "ground". The "deflection phase" q r s is emphasized with a rectangle, whereas the "baseline phase" s t p q is indicated with a hatched bar. A time domain switch allows to route the respective potentials to either the deflection node 8 or the virtual reference node 7. In another preferred embodiment, the t and p waves are excluded from the baseline phase as well.

Fig. 2D shows the typical frequency domain of potential changes in an ECG signal power spectrum. The frequency domain allows the use of filters in order to route the respective potentials to either the deflection node 8 or the virtual reference node 7. These filters and switches as in Fig. 2B can be combined sequentially and / or in parallel. Typical noise contamination in the 50 Hz or 60 Hz range due to power lines (not shown) can be separated and / or suppressed.

## Claims

1. A device (11) for detecting or monitoring electrical parameters in a human subject or animal subject by measuring electrical potentials, comprising
- at least one electrical connection attachable to tissue of said human or animal subject, which serves as a material electrode (5),
- at least one electronic circuitry and/or at least one microprocessor/microcontroller and, where appropriate, software, creating a node (7) which serves as a virtual reference electrode, wherein said node (7) can be held at an electrical reference potential by said circuitry and/or microprocessor/microcontroller, and
- means to measure electrical potential changes between said electrical reference potential of the node (7) and the electrical potential of the connection (5) at the tissue of said human or animal subject.

2. Device according to claim 1, wherein said electronic circuitry or said microprocessor/microcontroller comprises at least one capacitor (9) separating said electrical connection (5) and said node (7).

3. Device according to claim 1 or claim 2, wherein said electronic circuitry or said microprocessor/microcontroller comprises filters and/or switches for distributing signals resulting from electrical potential changes, in particular for distributing signals between the terminal (8) of the capacitor (9) which is assigned to the electrical connection (5) and the terminal of the capacitor which is assigned to the node (7).

4. Device according to one of the preceding claims wherein said means for measuring electrical potential changes are capable to measure a voltage change and/or a charge movement and/or a current.

5. Device according to one of the preceding claims, wherein only one electrical connection (5) to tissue of said human or animal subject is provided.

6. Device according to one of the preceding claims, in particular according to claim 5, wherein said electrical connection which is attachable to the tissue of said human or animal subject, comprises at least two contact points or contact areas.

7. Device according to claim 6, wherein said contact points or contact areas are provided at a distance of between at least 0,1 mm and at most 45 mm, wherein said distance preferably is between 5 mm and 25 mm, in particular between 10 mm and 15 mm.

8. Device according to one of the preceding claims, further comprising at least one data transmitter/data receiver, in particular at least one wireless data transmitter/data receiver.

9. Device according to one of the preceding claims, wherein the device is formed as or is integrated in an item of personal use to be worn by the subject.

10. Device according to one of the preceding claims, wherein the electrical parameters detected or monitored in a human subject or animal subject are related to cardiovascular conditions or related to cardiovascular diseases.

11. Item of personal use to be worn by a human subject or animal subject, **characterized in that** a device for detecting or monitoring electrical parameters by measuring electrical potentials, as defined in one of the preceding claims, forms this item or is integrated into this item.

12. A method for detecting or monitoring electrical parameters in a human subject or animal subject, in particular by using the device (11) according to one of claims 1 to 10, wherein
- at least one electrical connection (5) is attached to tissue, in particular to skin or mucous membrane of said subject, serving as a material electrode,
- a node (7) serving as a virtual reference electrode is created by at least one electronic circuitry and/or at least one microprocessor/microcontroller, and, where appropriate, software, wherein said node can be held at an electrical reference potential by said circuitry and/or microprocessor/microcontroller, and
- electrical potential changes are measured between said electrical reference potential of the node (7) and the electrical potential of the connection (5) at the tissue of said human or animal subject.

13. Method according to claim 12, wherein said electrical potential changes are measured as a voltage change and/or as a charge movement and/or as a current.

14. Method according to claim 13, wherein at least one capacitor (9), in particular at least one resistor (6) and at least one capacitor (9) connected in series, are used for the measurement of electrical potential changes, wherein preferably a charge movement towards said capacitor is detected measuring the voltage drop across the resistor (6).
